# EUROPEAN PATENT APPLICATION

(11) **EP 4 338 673 A1**
(43) Date of publication of application: **20.03.2024**
(21) Application number: 22195311.0
(22) Date of filing: 13.09.2022
(51) Int. Cl.: A61B 6/00

(54) **DETERMINATION OF A SOFT TISSUE-RELATED PROPERTY FROM X-RAY IMAGING DATA**

(71) Applicant: Koninklijke Philips N.V., 5656 AG Eindhoven (NL)
(72) Inventor: VON BERG, Jens, Eindhoven (NL); STEWART, Matthew Young, Eindhoven (NL); BRUECK, Heiner Matthias, 5656AG Eindhoven (NL); BYSTROV, Daniel, 5656AG Eindhoven (NL); KROENKE-HILLE, Sven, Eindhoven (NL); GOOßEN, Andre, Eindhoven (NL); HARDER, Tim Philipp, Eindhoven (NL)
(74) Representative: Philips Intellectual Property & Standards

(57) **Abstract**

Provided is an apparatus (100) for determining a soft tissue-related property of a bone constellation from X-ray imaging data. The apparatus (100) comprises a data processor (110) configured to obtain the X-ray imaging data of a region of interest including the bone constellation, and to determine, by utilizing a 3D pose estimation computer model (CM) into which the X-ray imaging data is fed, a pose estimation of the bone constellation, and at least one geometric parameter (d, t, r) defined between bones of the bone constellation, to determine the soft tissue-related property of the bone constellation.

## Description

### FIELD OF THE INVENTION

The present invention relates to medical imaging, and in particular to an apparatus and a computer-implemented method for determining a soft tissue-related property of a bone constellation from X-ray imaging data, and a computer program element.

### BACKGROUND OF THE INVENTION

In medical imaging, automatic, i.e. computer-aided, bone-fracture detection is widely addressed because bones are clearly visible in, e.g. musculoskeletal X-ray images. In general, the X-ray technique only allows to clearly visualize dense objects, such as bones. However, if less dense objects are to be examined by imaging, the data required for this are not explicitly visible in X-ray imaging data and thus non-bone-related or soft tissue injuries or abnormalities may not be directly observed or diagnosed therefrom. Rather, these may only be observed or diagnosed indirectly and therefore only by an experienced physician, which does not allow automation by computer.

Therefore, more time-consuming or cost-intensive image acquisition techniques, such as magnetic resonance tomography (MRT), are generally indicated for the purpose of observing or diagnosing soft tissue-related injuries or abnormalities. However, this requires appropriate equipment, skilled personnel and patience of the patient, etc.

### SUMMARY OF THE INVENTION

There may, therefore, be a need for means for automatic analysis of less dense objects in X-ray images. The object of the present invention is solved by the subject matter of the independent claims, wherein further embodiments are incorporated in the dependent claims.

According to a first aspect, there is provided an apparatus for determining a soft tissue-related property of a bone constellation from X-ray imaging data. The apparatus comprises at least one data processor configured to: (i) obtain the X-ray imaging data of a region of interest including the bone constellation; and (ii) determine, by utilizing a 3D pose estimation computer model into which the X-ray imaging data is fed, a pose estimation of the bone constellation, and at least one geometric parameter defined between bones of the bone constellation, to determine the soft tissue-related property of the bone constellation.

This allows automatic analysis of less dense objects in X-ray imaging data, i.e. on the basis of X-ray imaging data alone. In particular, this allows the X-ray imaging data to be analyzed by means of a processor, i.e. a computer, for bone constellation-related properties, especially at least one soft tissue-related property, that are not directly visible in an X-ray image. This automatic analysis significantly saves costs because no MRI is necessary. Further, automatic analysis requires less welltrained personnel, and the result of the analysis may be further processed computationally, documented automatically, etc.

The inventors have found, in a non-obvious way, that by considering or encoding at least one additional geometric parameter defined between bones of the bone constellation, an important source of information is available to perform soft tissue-related analyses and/or diagnoses beyond purely bone-related diagnoses, such as bone-fracture detection, from X-ray imaging data alone.

For example, the 3D pose estimation computer model may also be referred to as an extended and/or a parametrized 3D articulation model, wherein the 3D pose estimation computer model is configured to estimate pose parameters of the bone constellation, which may be a joint, e.g. an ankle joint or the like, etc. The 3D pose estimation computer model is parameterized with the additional geometric parameter described herein. The term pose as used herein may refer the projection geometry (i.e. pose in a narrow sense including the viewing direction and detector position), the joint-posture (i.e. flexion parameters), or the like. The basic 3D pose estimation computer model may comprise two modules or stages, wherein, in a first stage, pose-discriminative features (the bone silhouettes plus some inner contours) are detected by a convolutional neural network (CNN) as binary segmentation masks, and in a second, another CNN regresses all pose parameters from these feature masks. An advantage of this approach is that both components can be trained independently of each other, such that no pose groundtruth labels are required for the X-ray images. An exemplary 3D pose estimation model, not limiting the aspects described, that can be extended by the additional geometric parameter described herein is described in "Krönke et al. CNN-based pose estimation for assessing quality of ankle-joint X-ray images, SPIE Medical Imaging 2022", the content of which in its entirety is incorporated herein by reference.

For example, feature detection of the 3D pose estimation model may be implemented as follows: As a pre-processing step for the feature-detection network, a region of interest (ROI) may be automatically extracted from a given input X-ray image. This ROI-cropping approach may be based on a foveal fully convolutional network (FNet) predicting bone contours on a down sampled X-ray image and a probabilistic anatomical atlas of these bone contours. The detected bone contours may be registered to the atlas using a similarity transformation with an additional flipping degree-of-freedom for compensating the left /right laterality. The resulting transformation can be used to map an anatomically defined ROI of the joint from the atlas into the input image and to resample the thereby determined ROI. The feature detection network may be trained on this ROI resampled with high spatial resolution in order to sufficiently resolve the spatial constellation of contours being predictive for the 3D pose. For e.g. lateral (LAT) ankle X-ray images, an FNet may be trained to segment the outer silhouette of the fibula as well as selected salient contours of the tibia, talus and calcaneus bones. Contour-wise softmax layers may be attached to the FNet architecture. Thereby, the network is configured to predict a heatmap for each target contour and its complement, which are condensed to a binary prediction by applying a pixel-wise argmax operation on each of these heatmap pairs allowing for spatial co-existence of different contour classes. The training data may be generated by manually or automatically annotating structures in a number of X-ray images, e.g. a plurality of X-ray images, such as multiple, dozens, hundreds, etc., and discretized by dilating the contour annotations on the pixel the ROI. Thereby, the feature-detection tasks may be formulated as a standard segmentation problem.

For example, pose estimation of the 3D pose estimation model may be implemented as follows: In order to train the pose-estimation network without requiring X-ray images with pose groundtruth, an articulated 3D model of the bone constellation, e.g. an ankle joint may be constructed from e.g. a sequence of MR images obtained with varying flexion angle. Semi-manual segmentation of e.g. a tibia, fibula, talus and calcaneus bones in one MR image enabled creation of a surface-model represented using a triangular mesh. For each triangle of the mesh, features for model-adaptation may be trained using the image intensity and the resulting model was used to delineate the corresponding bone surfaces in the other MR images of the sequence. Bone-wise rigid transformations may be applied for the model-based segmentation, since all images were created with the same volunteer. By measuring the angle between the main axis of the tibia and that of the calcaneus, a flexion angle δ may be assigned to each MR image. Smooth interpolation of the surface models with respect to δ enables the mesh constellation to be estimated at any flexion angle between approx. 80° and approx. 135°. In order to generate synthetic projection data from this articulated 3D model, some pose parameters are defined. The central beam (or viewing direction) is determined by the angle θ with respect to the head-feet axis and the angle ϕ with respect to medial-lateral axis in the transverse plane. In order to simplify the sampling of the poseparameter for training of the pose-estimation network, detector and source may be assumed to rotate in a fixed C-arm-like configuration about a joint center, which means that ϕ and θ uniquely determine the position of the source and the detector. Here, the source-detector distance as well as joint-detector distance may be chosen to be within realistic regimes for clinical ankle lateral exams, and to approximately match the ROI on which the feature-detection network operates. The pose-parameters may include in-detector-plane rotation by an angle γ, translation by a 2D vector t, scaling by a factor s as well as the flexion angle δ. This basic 3D pose estimation computer model is configured to determine not only the acquisition parameters, e.g. geometric relations between source, joint model and detector, but also some free parameters of the joint itself, such as a scaling parameter and a joint flexion angle. This can be used, for example, to analyze and/or assess whether the projection image of the joint has been acquired in the desired pose, and thus whether or not the image quality is sufficient to enable the desired diagnosis. This basic 3D pose estimation computer model is currently not used for medical diagnosis of the image.

It is noted that in the above basic 3D pose estimation computer model, the bones of the bone constellation, e.g. a joint, are constrained to have a fixed constellation relative to each other, which limits the ability of the 3D pose estimation computer model to be adapted to a given X-ray image were those model constraints are not given, for example, due to pathologically induced variation in the bone constellation.

Herein, it is proposed to extend the above (basic) 3D pose estimation computer model, by the additional free geometric parameter between the bones, and its matching to also determine soft tissue-related or relevant geometric parameters and to determine them from X-ray imaging data, i.e. an X-ray image, alone. In addition to the angles between bones, that may reflect typical joint movements, such as flexion, abduction, rotation, etc., to be found in the acquired pose, the present disclosure focusses on the at least one geometric parameter between the bones in 3D described herein. The additional geometric parameter is a valuable source of information in a variety of diagnostic tasks, not only for image quality assessment, but especially also for medical diagnosis of the image. In contrast to trying to perform dislocation detection in a 2D image alone is restricted on deviation (dislocation) in the image plane alone and is impacted by positioning errors whenever present. The apparatus, system and method described herein, may circumvent both limitations as it is based on pose position estimating in 3D.

In addition to the at least one geometric parameter, other relevant parameters may also be combined, for example, in linear fashion or a logistic regression.

The apparatus may be implemented in software that is executed by the data processor, in hardware, or in a combination of hardware and software. The 3D pose estimation model may be stored as a program element in a computer memory and executed by the data processor, or may be implemented in a chip.

As used herein, the bone constellation may be any arrangement of bones of a human or animal body, in the torso, skull, spine, arm, hand, leg, foot, etc. In particular, the bone constellation may form a joint or articulation of the body, such as the knee, elbow, hand, shoulder, etc., mapped in the 3D pose estimation model.

Further, as used herein, soft tissue may be understood as distinct from bone constellation or bones as an object of the body with lower density than the bones, due to which property the soft tissue is not or poorly or hardly visible in the X-ray imaging data. Related to the bone constellation, the soft tissue may comprise, for example, cartilage, ligaments, tendons, muscles, fascia, or the like. The soft tissue may extend or be located between, adjacent to, or at a (small) distance around the bones.

It is noted that the apparatus may further comprise a suitable data interface configured to receive the X-ray imaging data. The imaging data may be provided by a Picture Archiving and Communication System (PACS), an X-ray detector, or the like. Further, the apparatus may be configured to generate a report on the soft tissue-related property upon detection.

Optionally, the apparatus may be further configured to trigger an output of at least the soft tissue-related property. Output may be understood as any type of making available for further processing, such as data output for further processing by a data processor or computer, the mere output of the property for output or information to a user, etc. This may allow the determined soft tissue-related property to be used for, preferably automatic, medical diagnosis.

Optionally, the apparatus may be further configured to explain and/or visualize the soft tissue-related property in or based on the 3D pose estimation computer model. For example, the determined soft tissue-related property may be graphically illustrated and/or explained in a graphical representation of the 3D pose estimation computer model. It may be graphically highlighted therein, labeled in text form, etc.

Optionally, the apparatus may be further configured to, particularly automatically, determine, from the at least one soft tissue-related property, a soft tissue-related lesion and/or abnormality. This may include any type of dislocation in or of the bone constellation where injured soft tissue may be involved. For example, a dislocated (too far, too narrow) bone may indicate, for example, tear of ligament from trauma, ligament weakness (osteoarthritis risk), degeneration of cartilage (osteoarthritis), other bones to be fractured (trauma, osteoporosis), other muscles or tendons to be ruptured (trauma, overload), congenital anomaly, impact of pose (errors), or the like. Further, for example, there may be also injuries that are more difficult to be detected from an X-ray image than a dislocated foot. In trauma cases like motor bike or bike accidents often hand injuries are overlooked when focusing on other life-threatening injuries of abdomen and head. The apparatus, system and method described herein may be used to flag suspicious cases for early treatment once the patient is otherwise stabilized.

Optionally, the apparatus may be further configured to compare the geometric parameter and/or pose estimation to a corresponding threshold to determine the soft tissue-related lesion and/or abnormality. For example, the geometric parameter may be a distance, including relative distance, between two bones, which may have a normal value between a lower threshold value and an upper threshold value, so that an injury, abnormality, or the like, may be determined by comparing the geometric parameter to this range of normal values. Also, by comparing with the corresponding threshold, it may be determined whether the pose is correct during image acquisition, thus avoiding false diagnosis. For example, deviation from normal parameters or thresholds may serve as additional diagnostic information in a routine radiological service also on asymptomatic cases. They may indicate, for example, a congenital anomaly or give prognostic parameters like a risk score to develop osteoarthritis. Data bases of typical or normal bone distances may be provided and used in diagnosis so that each individual patient is assessed with respect to statistical values.

Optionally, the apparatus may be further configured to adapt the respective threshold in case of an implant present in or related to the bone constellation. For example following a trauma, bones may be fixed surgically using implants and other devices, which can both strongly constrain the possible parameter variations, as well as introduce parameter settings which are not typical for the normal anatomy (e.g. out of the learned range). Such situations may require additional constraints and/or conditions to be introduced on an ad-hoc / situation specific basis. In this regard, the apparatus may be configured to obtain information, e.g. from data input by a user, from patient data, by feature detection in the X-ray imaging data, or the like, about the implant or other bone-fixing device, and may take the implant's impact on the soft tissue into account. For example, it is conceivable that a relatively small distance between bones caused by the implant or a bone fusion is given a correction value in order to exclude a false diagnosis.

Optionally, the apparatus may be further configured to combine the at least one geometric parameter at least one geometric parameter and/or the soft tissue-related property with patient-related data to determine the soft-tissue-related lesion and/or abnormality. The patient-related data may comprise, for example, a patient's age, since depending on age, some findings are more likely or less likely, the patient's body mass index (BMI), since this may affect the distances between bones, etc. Pre-existing medical conditions of the patient, etc., may also be taken into account as additional parameters. This may make the automatic diagnosis more precise.

Optionally, the apparatus may be further configured to trigger an alert to be output, based on the determined soft-tissue-related lesion and/or abnormality. For example, a reader of images with limited specialist knowledge of e.g. MSK applications may be alerted to "incidental findings". Often, reader or diagnostic attention is drawn to major structural lesions such as large fractures or other osseous traumas, which can lead to more subtle (e.g. soft-tissue) lesions or abnormalities being overlooked. Here, an automated alert may be provided, and also for additional (secondary) findings to be automatically populated into reporting schemes.

Optionally, the 3D pose estimation computer model may be parameterized by parameters including one or more of a flexion angle of the bone constellation, a viewing direction of the bone constellation, an X-ray detector position relative to the bone constellation, a translation in an X-ray detector plane, a rotation in an X-ray detector plane, a scaling in an X-ray detector plane, and the geometric parameter, wherein the 3D pose estimation computer model is trained to regress the one or more parameters.

Optionally, the at least one geometric parameter may comprise one or more of a distance between the bones, an orthogonal translation and a rotation. For example, the distance between bones, such as a talo-tibial distance, encoded in the 3D pose estimation computer model as an additional free parameter is an important source of information for the diagnosis of the patient. The distance between bones may reflect the size of a joint gap, such as the upper ankle joint gap, or the like. Thereby, a low distance value may indicate, for example, cartilage degeneration by age or osteoarthritis, an extra ordinarily high number may indicate tear of ligament and foot dislocation.

Optionally, the apparatus may be further configured to determine an axis between two neighboring bones of the bone constellation and to measure a distance between the two neighboring bones along the axis. For example, the distance between bones, such as a talo-tibial distance, encoded in the 3D pose estimation computer model as an additional free parameter is an important source of information for the diagnosis of the patient.

Optionally, the at least one geometric parameter may comprise a distance between the bones, and a tree or graph is used to encode a number of distances between bones in the 3D pose estimation computer model, wherein the bones are vertices and the distances are modelled by edges.

Optionally, the at least one geometric parameter may be a variable parameter of the 3D pose estimation computer model. As mentioned above, in the basic 3D pose estimation computer model, the bones of the bone constellation is constrained to have fixed constellation relative to each other. According to this example, the geometric parameter is a freely configurable parameter that can be varied. This may indicate the soft tissue related property.

According to a second aspect, there is provided a computer-implemented method for determining a soft tissue-related property of a bone constellation from X-ray imaging data. the method comprises obtaining the X-ray imaging data of a region of interest including the bone constellation; and determining, by utilizing a 3D pose estimation computer model into which the X-ray imaging data is fed, a pose estimation of the bone constellation, and a relative distance between bones of the bone constellation which relative distance is assigned to at least one soft tissue-related property of the bone constellation.

The method may preferably be performed by use of the apparatus according to the first aspect.

According to a third aspect, there is provided a computer program element, which when executed by a processor is configured to carry out the method of the second aspect, and/or to control an apparatus according to the first aspect.

According to a fourth aspect, there is provided a computer-readable storage or transmission medium, which has stored or which carries the computer program element according to the fourth aspect.

It is noted that the above examples and embodiments may be combined with each other irrespective of the aspect involved. Accordingly, the method may be combined with structural features of the apparatus of the other aspects and, likewise, the apparatus may be combined with features of each other, and may also be combined with features described above with regard to the method. These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig. 1 shows in a block diagram an exemplary apparatus for determining a soft tissue-related property of a bone constellation from X-ray imaging data.
Fig. 2 illustrates in subfigures 2A to 2F model parameters of an exemplary 3D pose estimation computer model.
Fig. 3 shows an example graph encoding neighboring bones in an exemplary bone constellation.
Fig. 4 shows in subfigures 4A and 4B in a respective X-ray image a geometric parameter for determining a soft tissue-related property of a bone constellation from X-ray imaging data.
Fig. 5 illustrates in a flow chart a method for determining a soft tissue-related property of a bone constellation from X-ray imaging data.

### DETAILED DESCRIPTION OF EMBODIMENTS

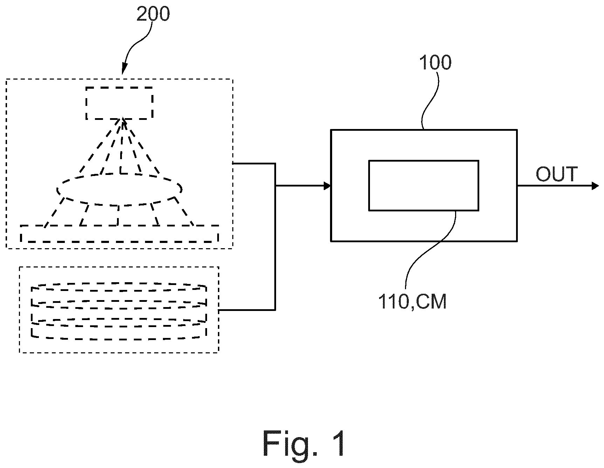

Fig. 1 shows in a schematic block diagram an exemplary apparatus 100 for determining a soft tissue-related property of a bone constellation from X-ray imaging data. The apparatus 100 may be any suitable computer and comprises a data processor 110, which is operatively connected to a suitable data interface and which may be operatively connected to further computer periphery. The x-ray imaging data may be provided as input data to the apparatus 100, particularly the data processor 110, by a data source 200, such as an X-ray detector, a Picture Archiving and Communication System (PACS), or the like. Output data OUT of the data processor 110 may be provided for further processing to another computer device, software module, an output device, e.g. a display, report system, or the like, the PACS, or the like.

The data processor 110 is configured to obtain the X-ray imaging data of a region of interest (ROI) including the bone constellation. These X-ray imaging data may be obtained from the data source 200. Further, the data processor 110 is configured to utilize a 3D pose estimation computer model CM, into which the obtained X-ray imaging data is fed or input, to determine a pose estimation of the bone constellation, and at least one geometric parameter defined between bones of the bone constellation, to determine the soft tissue-related property of the bone constellation.

The soft tissue-related property may include any soft tissue in, at, and/or surrounding the bone constellation included in the ROI, i.e. the X-ray imaging data (input data). In relation to the bone constellation, the soft tissue may comprise, for example, cartilage, ligaments, tendons, muscles, fascia, or the like. The soft tissue may extend or be located between, adjacent to, or at a (small) distance around the bones.

The at least one geometric parameter may include one or more of a distance between the bones, an orthogonal translation, and a rotation. The at least one geometric parameter is a variable parameter of the 3D pose estimation computer model CM.

The 3D pose estimation computer model CM may be a computer program element, implemented in the apparatus 100, stored in a computer memory, etc., and may be executed by the data processor 110.

Further, the apparatus 100, e.g. the data processor 110, may be configured to determine, from the at least one soft tissue-related property, a soft tissue-related lesion and/or abnormality. For determining the soft tissue-related property and/or to determine the soft tissue-related injury, lesion and/or abnormality, the apparatus 100 may be further configured to compare the geometric parameter and/or pose estimation to a corresponding threshold. The apparatus 100, e.g. the data processor 110, may be further configured to output the determined at least one the soft tissue-related property, and/or a determined injury, lesion and/or abnormality. The apparatus 100 may be further configured to trigger an alert to be output, based on the determined soft-tissue-related lesion and/or abnormality.

Optionally, the apparatus 100 may be further configured to explain and/or visualize the soft tissue-related property in or based on the 3D pose estimation computer model CM. Further, for determining the at least one soft tissue-related, the apparatus 100 may be further configured to determine an axis between two neighboring bones of the bone constellation and to measure a distance between the two neighboring bones along the axis, as one example of the at least one geometric parameter. Further, for determining the at least one soft tissue-related, the apparatus 100 may be further configured to adapt the respective threshold in case of an implant present in or related to the bone constellation. Optionally, the apparatus 100 may be further configured to combine the at least one geometric parameter at least one geometric parameter and/or the soft tissue-related property with patient-related data to determine the soft-tissue-related lesion and/or abnormality.

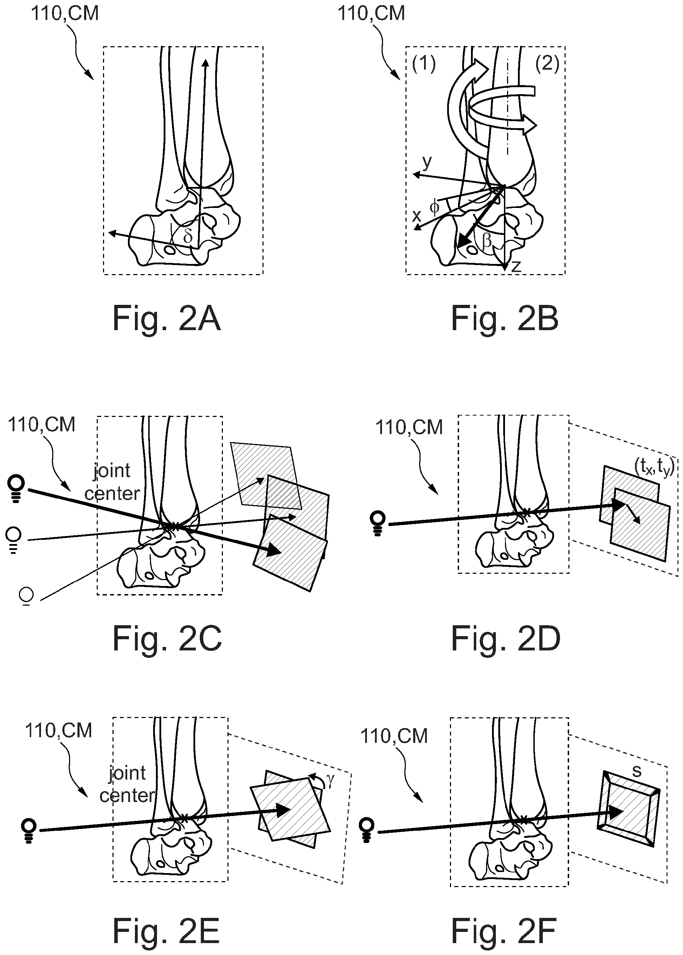

Fig. 2 illustrates in subfigures 2A to 2F exemplary model parameters of an exemplary 3D pose estimation computer model CM that is utilized by the data processor 110. This is only an exemplary selection of possible model parameters, wherein not all model parameters must necessarily be considered or encoded. It is noted that the at least one geometric parameter as explained above is an additional free parameter to the model parameters shown in Figs. 2A to 2F. Each of Figs. 2A to 2F is an example of an ROI including a bone constellation, which is here joint, especially an exemplary ankle joint. The 3D pose estimation computer model CM may also be referred to as a 3D articulation model.

The model parameter of Fig. 2A is a flexion angle. Fig. 2B illustrates the model parameter of a viewing direction, in which (1) indicates an azimuthal rotation and (2) indicates a polar rotation. The model parameter illustrated in Fig. 2C is a detector position. Fig. 2D illustrates the model parameter of a translator in detector plane. The model parameter of Fig. 2E is a rotation in detector plane. Fig. 2F illustrates the model parameter of scaling in detector plane, wherein the scaling may comprise, for example, indicating changing the joint size or the image sampling.

The 3D pose estimation model CM is trained to regress the model parameters considered or encoded therein. For example, such training of the 3D pose estimation model CM may be performed by using artificially generated images by using a 3D articulation shape model and its possible poses. This is not limited herein, since the training data may also be generated in a different way.

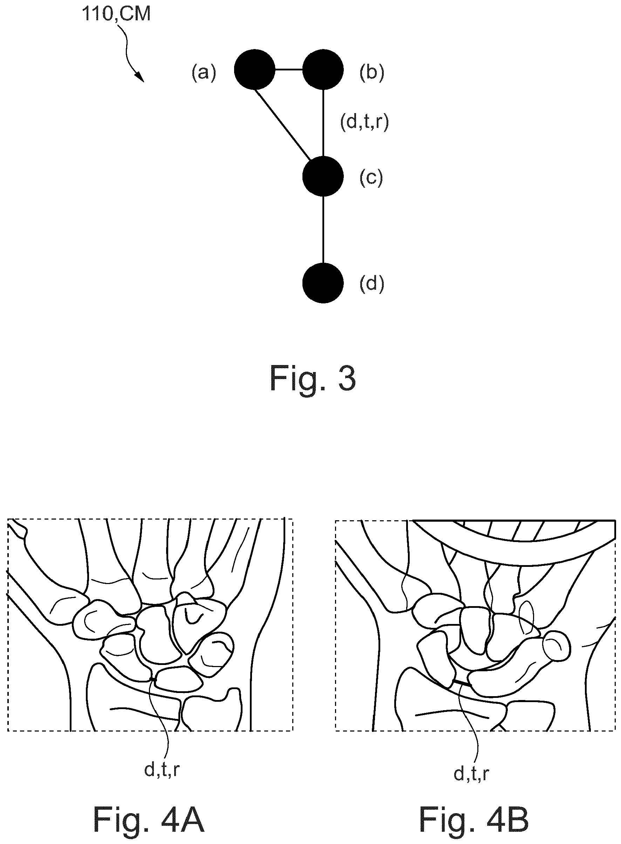

Fig. 3 shows an example graph encoding neighboring bones in an exemplary bone constellation. In the example shown in Fig. 3, which shall only serve for better illustration, the bone constellation included in the ROI is again an ankle joint comprising a fibula (a), a tibia (b), a talus (c), and a calcaneus (d), which can be illustrated as the graph encoding these bones. For example, the at least one geometric parameter comprises one or more of a distance d between the bones, an orthogonal translation t and a rotation r. It is again noted that each of the geometric parameters of distance d, orthogonal translation and rotation r, is a model parameter that may be encoded in the 3D pose estimation model in addition to the model parameters shown in Figs. 2A to 2F.

With respect to the exemplary ankle joint, as an example of the bone constellation, introducing the talo-tibial distance d as an additional free parameter allows more accurate adaptation of the 3D pose estimation computer model to cases with different or extraordinary distances. Once this model is parametrized and adapted to the image, the talo-tibial distance d encoded in the model parameter(s) provides diagnostic valuable information. For example, the distance d may reflect the size of the upper ankle joint gap, wherein a low value may indicate cartilage degeneration by age or osteoarthritis, and a high value may indicate tear of ligament and/or foot dislocation.

For implementing the 3D pose estimation computer model CM with the additional geometric parameter, by way of example, a tree or graph may be used to encode a number of distances between bones in the 3D pose estimation computer model, wherein the bones are vertices and the distances are modelled by edges.

Further, by way of example, for determining the distance d, the apparatus 100, e.g. the data processor 110, may be configured to construct an axis between two neighboring bones and to measure the distance d along that axis.

Fig. 4 shows in subfigures 4A and 4B in a respective X-ray image a geometric parameter for determining the soft tissue-related property of the bone constellation from X-ray imaging data. In Figs. 4A and 4B, the distance d, the translation t and/or rotation r relate to a scapho-lunate widening that can be taken as an indicator for degenerative arthritis.

As described above, the apparatus 100 is configured to utilize the 3D pose estimation computer model CM, which is added by including at least one additional free parameter, i.e. the at least one geometric parameter, e.g. the distance d, the translation t and/or rotation r to determine the scapho-lunate widening as an example of the soft tissue-related property. Comparing the scapho-lunate widening to normal values, statistics, or the like, may be utilized by the apparatus 100 to determine an injury, lesion, abnormality, etc. in the soft tissue, although the soft tissue is not visible in the X-ray imaging data.

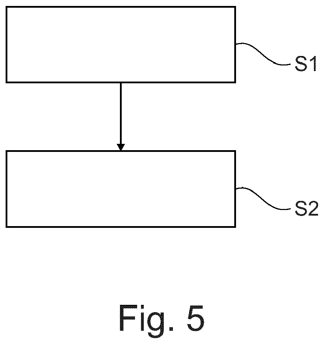

Fig. 5 illustrates in a flow chart a computer-implemented method for determining the soft tissue-related property of the bone constellation from X-ray imaging data. The method may be performed by the above apparatus 100. The method comprises, as step S1, obtaining the X-ray imaging data of a region of interest including the bone constellation, and, as step S2, determining, by utilizing the 3D pose estimation computer model CM into which the X-ray imaging data is fed, a pose estimation of the bone constellation, and a relative distance between bones of the bone constellation which relative distance is assigned to at least one soft tissue-related property of the bone constellation. As explained above, the at least one soft tissue-related property may be used for further diagnostic purposes, or the like.

According to a further aspect, there is provided a computer program element for controlling the X-ray imaging system of the third aspect, which, when being executed by processing unit, is adapted to perform the method steps of the fourth aspect.

According to a further aspect, there is provided a computer readable medium having stored in the computer program element of the fifth aspect.

A computer program element might therefore be stored on a computer unit, which might also be an embodiment of the present invention. This computing unit may be adapted to perform or induce performance of the steps of the method described above. Moreover, it may be adapted to operate the components of the above-described apparatus. The computing unit can be adapted to operate automatically and/or to execute orders of a user. A computer program may be loaded into a working memory of a data processor. The data processor may thus be equipped to carry out the method of the invention.

This exemplary embodiment of the invention covers both the computer program that has the intervention installed from the beginning, and a computer program that by means of an update turns an existing program into a program that uses the invention.

A computer program may be stored and/or distributed on a suitable medium, such as optical storage media, or a solid state medium supplied together with, or as a part of other hardware, but may also be distributed in other forms, such as via the Internet or other wired or wireless telecommunication systems. However, the computer program may also be presented over a network like the World Wide Web, and can also be downloaded into the working memory of a data processor from such a network.

According to a further exemplary embodiment of the present invention, a medium for making a computer program element available for downloading is provided, which computer program element is arranged to perform a method according to one of the previously described embodiments of the invention.

It should to be noted that embodiments of the invention are described with reference to different subject-matters. In particular, some embodiments are described with reference to method-type claims, whereas other embodiments are described with reference to device-type claims. However, a person skilled in the art will gather from the above, and the following description that, unless otherwise notified, in addition to any combination of features belonging to one type of subject-matter, also other combinations between features relating to different subject-matters is considered to be disclosed with this application.

All features can be combined to provide a synergetic effect that is more than the simple summation of the features.

While the invention has been illustrated and described in detail in the drawings and foregoing description, such illustration and description are to be considered illustrative or exemplary, and not restrictive. The invention is not limited to the disclosed embodiments.

Other variations to the disclosed embodiments can be understood, and effected by those skilled in the art in practicing the claimed invention, from a study of the drawings, the disclosure, and the dependent claims.

In the claims, the word "comprising" does not exclude other elements or steps, and the indefinite article "a" or "an" does not exclude a plurality. A single processor, or other unit, may fulfil the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SIGNS:

- 100: apparatus
- 110: data processor
- CM: 3D pose estimation computer model
- 200: imaging data source

- d, t, r: geometric parameter

## Claims

1. An apparatus (100) for determining a soft tissue-related property of a bone constellation from X-ray imaging data, the apparatus comprising a data processor (110) configured to:
obtain the X-ray imaging data of a region of interest including the bone constellation; and
determine, by utilizing a 3D pose estimation computer model (CM) into which the X-ray imaging data is fed, a pose estimation of the bone constellation, and at least one geometric parameter (d, t, r) defined between bones of the bone constellation, to determine the soft tissue-related property of the bone constellation.

2. The apparatus of claim 1, wherein the apparatus (100) is further configured to trigger an output of at least the soft tissue-related property.

3. The apparatus of claim 1 or claim 2, wherein the apparatus (100) is further configured to explain and/or visualize the soft tissue-related property in or based on the 3D pose estimation computer model.

4. The apparatus of any one of the preceding claims, wherein the apparatus (100) is further configured to determine, from the at least one soft tissue-related property, a soft tissue-related lesion and/or abnormality.

5. The apparatus of claim 4, wherein the apparatus (100) is further configured to compare the geometric parameter and/or pose estimation to a corresponding threshold to determine the soft tissue-related lesion and/or abnormality.

6. The apparatus of claim 5, wherein the apparatus (100) is further configured to adapt the respective threshold in case of an implant present in or related to the bone constellation.

7. The apparatus of claim 5 or 6, wherein the apparatus (100) is further configured to combine the at least one geometric parameter and/or the soft tissue-related property with patient-related data to determine the soft-tissue-related lesion and/or abnormality.

8. The apparatus of any one of claims 3 to 7, wherein the apparatus (100) is further configured to trigger an alert to be output, based on the determined soft-tissue-related lesion and/or abnormality.

9. The apparatus of any one of the preceding claims, wherein the 3D pose estimation computer model is parameterized by parameters including one or more of a flexion angle of the bone constellation, a viewing direction of the bone constellation, an X-ray detector position relative to the bone constellation, a translation in an X-ray detector plane, a rotation in an X-ray detector plane, a scaling in an X-ray detector plane, and the geometric parameter, wherein the 3D pose estimation computer model is trained to regress the one or more parameters.

10. The apparatus of any one of the preceding claims, wherein the at least one geometric parameter comprises one or more of a distance between the bones, an orthogonal translation and a rotation.

11. The apparatus of any one of the preceding claims, wherein the apparatus (100) is further configured to determine an axis between two neighboring bones of the bone constellation and to measure a distance between the two neighboring bones along the axis.

12. The apparatus of any one of the preceding claims, wherein the at least one geometric parameter comprises a distance between the bones, and a tree or graph is used to encode a number of distances between bones in the 3D pose estimation computer model, wherein the bones are vertices and the distances are modelled by edges.

13. The apparatus of any one of the preceding claims, wherein the at least one geometric parameter is a variable parameter of the 3D pose estimation computer model.

14. A computer-implemented method for determining a soft tissue-related property of a bone constellation from X-ray imaging data, comprising:
obtaining (S1) the X-ray imaging data of a region of interest including the bone constellation; and
determining (S2), by utilizing a 3D pose estimation computer model into which the X-ray imaging data is fed, a pose estimation of the bone constellation, and a relative distance between bones of the bone constellation which relative distance is assigned to at least one soft tissue-related property of the bone constellation.

15. A computer program element, which when executed by a processor is configured to carry out the method of claim 14.
